# EUROPEAN PATENT APPLICATION

(11) **EP 0 942 066 A2**
(43) Date of publication of application: **15.09.1999**
(21) Application number: 99104389.4
(22) Date of filing: 04.03.1999
(51) Int. Cl.: C12N 5/06, C12N 5/08, C12N 5/10, G01N 33/50, A61K 45/00

(54) **Cell line of blood-brain barrier**

(30) Priority: 04.03.1998 JP 5211898; 31.03.1998 JP 8751598
(71) Applicant: KYOWA HAKKO KOGYO CO., LTD., Chiyoda-ku, Tokyo 100-8185 (JP)
(72) Inventor: Obinata, Masuo, Sendai-shi, Miyaga 980-0871 (JP); Hosoya, Ken-ichi, Sendai-shi, Miyagi 982-0826 (JP); Terasaki, Tetsuya, Sendai-shi, Miyagi 981-3101 (JP)
(74) Representative: VOSSIUS & PARTNER

(57) **Abstract**

A brain capillary endothelial cell line having the ability of expressing Na⁺ independent glucose transport carrier (GLUT-1) and/or P-glycoprotein (mdr1a gene product); and an astrocyte cell line which improves the ability of expressing GLUT-1 of the brain capillary endothelial cell by co-culturing with the brain capillary endothelial cell of the above cell line.

## Description

### BACKGROUND OF THE INVENTION

### 1. Field of the Invention

The present invention relates to cell engineering technology in blood-brain barrier. In particular, the present invention relates to brain capillary endothelial cells constituting the blood-brain barrier or to a cell line having the characteristics of astrocyte cells. These cells can be used in screening to identify centrally acting drugs (anti-dementia drug, therapeutic agents for brain tumors, viral therapeutic agents, and drugs acting on nerves) based on the blood-brain barrier transmission mechanism and in screening to eliminate drugs having the problem of side effects in the nerve center. Furthermore, the present invention relates to a cell line which can be used in screening of drugs targeting various receptors expressed in brain capillary endothelial cells (brain microcirculation improving drugs, therapeutic drugs for brain edema). Moreover, the present invention relates to a cell line which can be used in screening of drugs targeting cytopathy (brain angiopathic dementia, Alzheimer's dementia) of brain capillary endothelial cells.

### 2. Brief Description of the Background Art

The capillary endothelial cells of the brain transport not only nutrients but also drugs to the brain via various transport systems expressed in cell membranes. The transport system is collocatively referred to as "the blood-brain barrier", e.g., a special physiological function which excretes metabolites of neurotransmitters and foreign materials from the brain to circulating blood although its physical existence is hardly elucidated. Evidence for the blood brain barrier is surmised from the knowledge that many drugs (foreign materials) are hardly transferred to the brain in spite of being highly lipid-soluble. The reason for this is considered to be in that drugs once transferred to the brain are excreted from the brain at dozens to hundreds as high as the transfer rate of the drugs. Accordingly, the blood-brain barrier plays a significantly important role in the transfer of a centrally acting drug. For example, the transfer of a therapeutic drug against AIDS virus, azidothymidine (AZT), into the brain is significantly limited due to the function of an excretion transport system in the blood-brain barrier [J. *Pharmacol. Exp. Ther., 281*: 369-375 (1997), *ibid. 282*: 1509-1517 (1997)].

For the reason described above, by development of the brain capillary endothelial cultured cell line, it is possible to conduct not only reasonable screening of centrally acting drugs (anti-dementia drug, therapeutic agents for brain tumors, viral therapeutic agents, and drugs acting on nerves) and drugs having the problem of side effects in the nerve center based on the blood-brain barrier transmission mechanism, but also evaluation of the effects of drugs targeting various receptors expressed in brain capillary endothelial cells (brain microcirculation improving drugs, therapeutic drugs for brain edema) and the effects of drugs targeting cytopathy (brain angiopathic dementia, Alzheimer's dementia) of brain capillary endothelial cells. In a conventional *in vitro* experimental system for the blood-brain barrier, experiments have been performed on bovine cells prepared according to the method of isolating primary-culture brain capillary endothelial cells (Audus & Bochardt et *al., Pharm.* Res., *3*: 81-87 (1986)). However, it was reported that the transfer rate of L-dopa transported in a neutral amino acid transport system and the transfer rate of glucose transported in a hexose transport system are lower by one tenth or less in the primary-culture cells than in *vivo* [Pardridge *et al., J. Pharmacol. Exp. Ther., 253:* 884-891 (1990)], and transmittance of materials by simple diffusion is higher by 150-fold or more in the bovine primary-culture cells than the in *vivo* system, thus making the primary-culture cells inadequate as a model of the blood-brain barrier.

A gene for P-glycoprotein known as an excretion system present in the blood-brain barrier is called mdr (multi drug resistance) 1, and mdr1a, mdr1b and the like are known. A gene for P-glycoprotein is expressed mainly in multiple drug resistant cancer cells but its expression in normal cells is also observed. It is considered that mdr1a which is one gene of P-glycoprotein is present in mouse brain capillaries [Schinkel, A.H. *et al., Cell,* 77, 491-502 (1994)]. Although MBEC-4 [Tatsuta, T. *et al., J. Biol. Chem., 267*: 20383-20391 (1992)] is exemplified as a brain capillary endothelial cell line, it is considered that such brain capillary endothelial cells are not normal because a mdr1a gene product expressed in *vivo* is not expressed in the MBEC-4 cell and P-glycoprotein (mdr1b gene product) not expressed in *vivo* is expressed.

On the other hand, it is known that when brain capillary endothelial cells are co-cultured with astrocyte cells, the ability of the brain capillary endothelial cells of expressing GLUT-1 is improved [Hayashi, Y. *et al., GLIA, 19:* 13-26 (1997)]. However, astrocyte cells used in co-culture are primary-culture astrocyte cells, so there are the problems that the cells should be prepared each time; also because the cells are usually separated and cultured from a rat one day after birth, they differ from astrocyte cells from mature rats (Swanson R.A. *et al., J. Neurosci., 17:* 932-940 (1997)). Although human-derived neuroglia tumor cells (glia cells), such as KG-1-C, U251, GI-1, and the like, and rat-derived tumorigenic glia cells, such as RCR-1, C6, and the like, have been used heretofore as cell lines, these glia cells express glial fibrillary acidic protein (hereinafter referred to as "GFAP") and have not only the properties of astrocytes but also the properties of oligodendrocytes forming a myelin sheath. Therefore, these cells do not accurately reflect the functions of only the original astrocytes. For example, in C6 cells, GLT1 and GLAST that are Na⁺-dependent L-glutamic acid transporters inherently present in astrocytes are not expressed, and EAAC1 that is a protein expressed in nerve cells is expressed [Palos, T.P. *et al., Mol. Brain. Res., 37:* 297-303 (1996)].

### SUMMARY OF THE INVENTION

An object of the present invention is to establish a cell line of the blood-brain barrier which can be used in screening of centrally acting drugs (anti-dementia drug, therapeutic agents for brain tumors, viral therapeutic agents, and drugs acting on nerves) based on the blood-brain barrier transmission mechanism, drugs targeting various receptors expressed in brain capillary endothelial cells (brain microcirculation improving drugs, therapeutic drugs for brain edema) or drugs targeting cytopathy (brain angiopathic dementia, Alzheimer's dementia) of brain capillary endothelial cells.

This and other objects of the present invention have been accomplished by a brain capillary endothelial cell line having the ability of expressing Na⁺ independent glucose transport carrier (GLUT-1) and/or P-glycoprotein (mdr1a gene product).

In another embodiment of this invention, this and other objects have been accomplished by an astrocyte cell line which improves the ability of the brain capillary endothelial cell of expressing GLUT-1 when co-cultured with the brain capillary endothelial cell.

### DETAILED DESCRIPTION OF THE INVENTION

This application is based on Japanese application Nos. 10-52118 filed on March 4, 1998 and 10-87515 filed on March 31, 1998, the entire contents of which are incorporated hereinto by reference.

Conventional methods of establishing a cell line include (1) a method of continuing culturing primary culture cells for a long time in order to yield cells that acquired an ability to grow infinitely, and (2) a method of introducing an oncogene, such as SV40 or the like, to primary culture cells to immortalize the cells. However, there are following disadvantages: in the method (1), desired cells cannot necessarily be obtained, and in the method (2), the oncogene is introduced exclusively in those cells which proliferate and there is a difference among those cells in chromosome introduction site. To overcome these problems, a method of obtaining a cell line by isolating cells from desired tissues in a transgenic mouse (to which an oncogene, such as SV40 or the like, has been introduced) was developed [Yanai, N. *et al., Exp. Cell. Res., 197:* 50-56 (1991); Yanai, N. et *al., Jpn. J. Cancer Res., 82:* 1344-1348 (1991)]. In this method, because an oncogene necessary for immortalization has been integrated in cells, a cell line can be established at considerably high frequency while maintaining differentiated traits.

The method of establishing the cell line of the present invention includes a method of isolating cells from desired tissues in a transgenic animal to which an oncogene has been introduced, and then culturing the cells to establish a cell line.

Examples of the oncogene to be introduced include a large T antigen gene of temperature-sensitive mutant SV40tsA58. Examples of the transgenic animals include transgenic mammals. Preferable transgenic mammals include transgenic mice and transgenic rats. The tissues may be any tissues removed from a desired cell line, and examples include brain.

A desired tissue is removed from the transgenic animals, and cells are separated and recovered by an enzymatic method [Ichikawa, N. *et al., J. Pharmacol. Toxicol. Method., 36*: 45-52 (1996); Goldstein, G.W. *et al., J. Neurochem.,* 25: 715-717 (1975); Meyer, J. et *al., J. Neurochem., 57*: 1971-1977 (1991)]. After culturing, cloning of the cells is conducted according to, e.g., a colony formation method *[Endocrinology, 136*: 4084-4091 (1995)], from which a cell line can be established by repeating cloning several times.

In the present invention, a brain capillary endothelial cell line and an astrocyte cell line were established from transgenic mice and transgenic rats, respectively, to which a large T antigen gene from temperature sensitive mutant SV40tsA58 has been introduced. Examples of the brain capillary endothelial cell line include TM-BBB1, TM-BBB2, TM-BBB3, TM-BBB4, and TMBB5. Examples of the astrocyte cell line include TR-AST32, TR-AST811, TR-AST912, TR-AST932, and TR-AST943.

The present invention relates to the use of the cell lines described for screening to identify centrally acting drugs; screening to eliminate drugs having the problem of side effects in the nerve center; screening of drugs targeting various receptors expressed in brain capillary endothelial cells; or screening of drugs targeting cytopathy.

The invention further relates to a method for producing a pharmaceutical composition comprising screening to identify centrally acting drugs; screening to eliminate drugs having the problem of side effects in the nerve center; screening of drugs targeting various receptors expressed in brain capillary endothelial cells; or screening of drugs targeting cytopathy, and formulating said drug identified by the described screening methods into a pharmaceutical composition. The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions etc. Compositions comprising drugs identified by the above screening methods and a pharmaceutically acceptable carrier can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitcred by periodic assessment. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

The method of confirming expression of GLUT-1 in the brain capillary endothelial cell line includes Western blotting using anti-mouse GLUT-1 monoclonal antibody. The method of confirming the expression of mdr1a mRNA includes Northern blotting using a probe for mdr1a [Hsu, S.I. *et al., J. Biol. Chem., 264:* 12053-12062 (1989)]. Furthermore, the method of measuring the rate of glucose transport includes analysis of the incorporation of radio-labeled glucose into the cell line.

The method of confirming expression of glial fibrillary acidic protein (GFAP) which is a protein unique to the astrocyte cell line includes immunostaining. Furthermore, the glutamic acid transporter ability of Na⁺ dependent L-glutamic acid transporter is confirmed by analysis of the incorporation of [³H]L-glutamic acid ([³H]L-Glu]) into the cell line.

The present invention will now be illustrated in greater detail with reference to Examples, but it should be understood that the present invention is not construed as being limited thereto.

### EXAMPLES

### Example 1

### Establishment of Mouse Cultured Brain Capillary Cell Line:

Brains were removed from 7-week-old transgenic mice (3 animals) to which a large T antigen gene from temperature-sensitive mutant SV40tsA58 had been introduced. Separation and recovery of the brain capillary endothelial cells was conducted using an enzymatic method in the following manner. The brains were placed in a Petri dish containing a separation buffer [122 mM NaCl, 3 mM KCl, 1.4 mM CaCl₂, 1.2 mM MgSO₄, 0.4 mM K₂HPO₄, 25 mM NaHCO₃, 10 mM glucose, 10 mM HEPES (pH 7.4)] which had been sterilized at 121°C for 15 minutes, transferred to a clean bench and washed with the separation buffer. The brains were cut into square pieces with a side of 2 mm or finely divided with scissors, introduced into a Potter-Elvehjiem homogenizer and homogenized 10 times in 4-fold volume excess of a homogenization buffer [10 mM Tris-HCl (pH 7.4), 1 mM EDTA, 250 mM sucrose, 1 mg/ℓ leupeptin, 1 mM pheylmethylsulfonyl fluoride (PMSF)]. Furthermore, an equal volume of 32% dextran solution was added thereto to adjust the dextran concentration to 16%, and homogenized 3 times. The resulting brain homogenate was introduced into a centrifuge tube and centrifuged at 4,500×g at 4°C for 15 minutes. The lipid layer was transferred to another centrifuge tube and centrifuged again at 4,500×g at 4°C for 15 minutes. The first precipitate and the precipitate from the centrifuged lipid layer were introduced into one centrifuge tube, an equal volume of the homogenization buffer and 32% dextran solution were added thereto, and the mixture was stirred and centrifuged at 4,500×g at 4°C for 15 minutes. To the resulting pellet, 0.1% collagenase/disperse solution was added, and shaken at 37°C at 100 rpm for 3 hours in a water-bath shaker. Thereafter, it was centrifuged at 200×g, at 4°C for 10 minutes to recover the cells as a pellet.

The resulting cells were cultured for 3 days in a GM-A medium [20 mM NaHCO₃, 2 mM L-glutamine, 15 mg/ℓ endothelial cell growth factor (ECGF) , 50 IU/mℓ benzylpenicilline potassium, 50 mg/ℓ streptomycin sulfate, 2.5 mg/ℓ amphotelicin B, 10 U/mℓ heparin, 20% fetal bovine serum, 9.6 g Dulbecco modified Eagle medium (hereinafter referred to as "DMEM"), filled up to 1 ℓ by adding distilled water twice] in a 5% CO₂ incubator at 37°C. On the 4th day, the medium was exchanged for a GM-B medium (20 mM NaHCO₃, 2 mM L-glutamine, 15 mg/ℓ ECGF, 50 IU/mℓ benzylpenicilline potassium, 50 mg/ℓ streptomycin sulfate, 10% fetal bovine serum, 9.6 g DMEM, filled up to 1 ℓ by adding distilled water twice) in a 5% CO₂ incubator at 33°C. Thereafter, the medium was exchanged for a fresh medium every 3 days. Culturing was conducted using a 35 mm culture dish coated with N-isopropyl acrylamide monomer (IPAAm) treated with 2 mℓ fibronectin at 37°C for 1 hour. The 35 mm culture dish coated with IPAAm was washed with distilled water which had been added to a polystyrene culture dish, had been exposed with 0.25 Mgy electron beam (200 kv, under 1.3×10⁻⁴ Pa) by means of an area beam electron processing system (Nisshin High Voltage, Kyoto) and then had been cooled, was dried with a nitrogen gas, was sterilized with an ethylene oxide gas, and then used for culturing. For subculture, the cells were treated with 0.1% collagenase/disperse solution at 37°C for 5 minutes, allowed to stand at 4°C for 2 hours, and removed from the culture dish.

Cloning of the cells was conducted by a colony formation method in the following manner. The cells after subculture for 3 generations were recovered, and 1×10³ cells were plated onto a 90 mm culture dish to form a colony. The medium was removed under suction, and a cloning cup with grease applied to one side was set up in the culture dish such that the colony was placed at the center. Into the cup only, 0.25% trypsin-EDTA solution was added, and incubated at 33°C for 1 minute. The GM-B medium was added into the cup, and the cells were removed by pipetting and cultured in a culture dish. This operation was conducted twice to give cell lines. The cell lines were proliferated with a doubling time of about 20 hours and even if subculture proceeded till about 20 generations, the cells were stable without any significant alternation in their proliferation ability. They were each designated TM-BBB1, TM-BBB2, TM-BBB3, TM-BBB4, and TM-BBB5.

TM-BBB4 has been deposited as FEPM BP-6282 since March 3, 1998 under the Budapest Treaty with National Institute of Bioscience and Human-Technology, Agency of Industrial Science and Technology (1-3, Higashi 1-chome, Tsukuba-shi, Ibaraki, 305-8566, Japan).

### Example 2

### Expression of GLUT-1 in Brain Capillary Endothelial Cell Line and Ability of Transporting Glucose:

Expression of GLUT-1 in the cells lines obtained in Example 1, that is, TM-BBB1, TM-BBB2, TM-BBB3, TM-BBB4 and TM-BBB5, was examined by Western blotting. With respect to the protein of GLUT-1, 3 mg brain capillary fraction and the cells cultured in a 10 cm dish were washed twice with PBS and scraped with 6 mℓ of the sonication buffer (250 mM sucrose, 100 mM sodium phosphate, 5 mM EDTA, 1 mM PMSF, 25 mM benzamide, 50 mM NaF, pH 7.4). It was sonicated 3 times for 30 seconds under cooling on ice, and the lysate was centrifuged at 550xg for 10 minutes to remove nuclei and undisrupted cells, and the supernatant was centrifuged at 100,000×g for 1 hour at 4°C. The pellet was lyzed at 4°C overnight under gentle stirring in a membrane buffer (50 mM Tris-HCl, 1 mM EDTA, 1 mM PMSF, pH 7.4) containing 2% Triton X-100, and centrifuged at 100,000×g to give a supernatant as a sample. With respect to GLUT-3, 2×10⁶ cells of TM-BBB and 3 mg brain capillary fraction were each homogenized in 5 mℓ homogenization buffer, centrifuged at 900×g at 4°C for 10 minutes, and the supernatant was further centrifuged at 110,000×g at 4°C for 75 minutes. The resulting pellet was added to 0.5 mℓ lysing buffer [10 mM Tris-HCl (pH 7.4), 1 mM EDTA, 0.5% Triton X-100, 0.5% sodium deoxycholate, 1 mM PMSF] and lyzed, and insoluble matters were centrifuged at 14,000×g at 4°C for 10 minutes to remove precipitates, and the supernatant was used as a sample. The sample was subjected to SDS polyacrylamide gel electrophoresis to separate a protein. The gel concentration was 10%. Transfer of the protein from the gel to a nitrocellulose membrane was conducted in a semi-dry type transfer unit [Towbin, H. *et al., Proc. Natl. Acad. Sci. USA, 76:* 4330-4354 (1979)]. Anti-mouse GLUT-1 antibody or anti-mouse GLUT-3 antibody was placed on a transfer membrane and incubated at room temperature for 1 hour and washed 3 times with a rinse buffer (0.1% Tween 20, PBS). The protein on the transfer membrane was then incubated for 1 hour with peroxidase-labeled anti-rabbit IgG as the secondary antibody at room temperature. It was washed for 3 days with the rinse buffer and then colored with ECL Western blotting detection reagents (Amersham). The results are shown in Table 1.

**Table 1**

| | TM-BBB1 | TM-BBB2 | TM-BBB3 | TM-BBB4 | TM-BBB5 | Brain capillary fraction |
|---|---|---|---|---|---|---|
| GLUT-1 | + | ++ | ++ | ++ | ++ | +++ |
| GLUT-3 | - | - | - | - | - | - |
| +++: strong expression ++: expression +: weak expression -: no expression | | | | | | |

A band indicating the expression of GLUT-1 was confirmed in any of TM-BBB1, TM-BBB2, TM-BBB3, TM-BBB4 and TM-BBB5 similar to the brain capillary fraction. On the other hand, the expression of GLUT-3 was not confirmed in any of TM-BBB1, TM-BBB2, TM-BBB3, TM-BBB4 and TM-BBB5. GLUT-1 is specifically expressed in brain capillary endothelial cells, while GLUT-3 is expressed in brain parenchymal cells, so it was confirmed that any of the cell lines are brain capillary endothelial cells.

The glucose transfer ability of the cell lines obtained in Example 1, that is, TM-BBB1, TM-BBB2, TM-BBB3, TM-BBB4 and TM-BBB5, were examined in terms of the incorporation of 3-O-methyl-D-glucose (3-OMG). Each of TM-BBB's was plated onto a 24-well cell culture plate at a density of 3×10⁵ cells/well/mℓ medium and cultured in a CO₂ incubator at 33°C for 24 hours to make the cells confluent.

The incorporation of 3-OMG was measured in the following manner. First, the medium was suctioned, and the cells were washed with an uptake buffer at 37°C [a solution containing 122 mM NaCl, 3 mM KCl, 1.4 mM CaCl₂, 1.4 mM MgSO₄(7H₂O), 0.4 mM K₂HPO₄, 10 mM glucose, 10 mM HEPES and 25 mM NaHCO₃, bubbled with 5% CO₂/95% O₂ for 20 minutes and adjusted to pH 7.4 with NaOH], and 0.2 mℓ of the uptake buffer containing 46.3 kBq/well [³H]3-OMG and 9.25 kBq/mℓ [¹⁴C]inulin at 37°C was added. Ten seconds later, the uptake buffer was removed, and the cells were washed with the uptake buffer at 4°C. Subsequently, the uptake buffer was removed after 20 seconds, 30 seconds, 1 minute, or 10 minutes, and the cells were treated in the same manner. For measurement of the dependence of 3-OMG incorporation on the substrate, the cells were washed with the uptake buffer at 37°C, and then 0.2 mℓ of the uptake buffer at 37°C containing 4.36 kBq/well [³H]3-OMG and 9.25 kBq/well [¹⁴C]inulin was added. However, the uptake buffer used contained 0.5, 1, 5, 10, 20, 30, and 50 mM unlabelled 3-OMG in place of 0.25, 0.5, 2.5, 5, 10, 15, and 25 mM NaCl, respectively. Twenty seconds later, the uptake buffer was removed, and the cells were washed with the uptake buffer at 4°C. Then, the cells were lyzed overnight with 1 mℓ PBS containing 1% Triton X-100 and then measured for radioactivity in a liquid scintillation counter.

The Km and Vmax for incorporation of 3-OMG were analyzed using a non-linear minimum square program [Yamaoka, K. *et al., J. Pharmacobio-Dyn, 4:* 879-885 (1981)] using a plot formula (V = Vmax x [S]/(Km + [S]); Vmax is a maximum rate constant, Km is a Michaelis constant, and [S] is a substrate concentration) for the rate of incorporation of 3-OMG. The results indicated that the incorporation of [³H]3-O-methyl-D-glucose ([³H]3-OMG) as the substrate of GLUT1 was concentration-dependent, the Michaelis constant (Km) was 6.6 mM, and the maximum incorporation rate constant (Vmax) was 44 nmol/min/mg protein. The clearance of incorporation of [³H]3-OMG (Vmax/Km) for the cell lines was 6.6 µℓ/min/mg protein.

An experiment of in *vivo* incorporation of glucose by use of the brain uptake index method reported Km = 6.67 mM, Vmax = 1562 nmol/min/g brain [*Neurochem. Res.*, *18:* 591-597 (1993)]. The cell lines established this time indicated a Km value close to the above-described experimental value, and it was confirmed that the characteristics of GLUT-1 are near in vivo. Furthermore, Vmax/Km, that is, the clearance of incorporation of 3-OMG was 6.6 µL/min/mg protein. Moreover, the *in vivo* incorporation clearance was 133.1 µL/min/mg protein, and it was confirmed that the cell lines have about 1/20 or less transport activity than *in vivo*. It has been reported that the function of GLUT-1 and P glycoprotein is reduced to about 1/300 by analysis in a bovine primary culture system [Pardridge W. M., *Peptide Drug Delivery to the Brain,* Raven Press, New York, p. 96 (1991)], so it was indicated that the cell lines maintain sufficiently high transport function.

### Example 3

### Expression of mdr1a in Established Brain Capillary Endothelial Cell Line:

Expression of mdr1a and mdr1b by cell line TM-BBB4 obtained in Example 1 ℓ was examined by Northern blotting.

RNA was separated from TM-BBB4 and large intestines of Slc:ddY mice as the control [Croop, *et al., Mol. Cell. Biol., 9*: 1346-1350 (1989)], and mRNA was obtained using an RNA purification kit (Pharmacia Biotech). The mRNA was subjected to electrophoresis at 5 pg/lane in 1% agarose gel. After blotted onto Hybond N⁺ nylon membrane, it was dried at 80°C for 2 hours. mdr1a and mdr1b probes used were cleaved from a plasmid (Hsu, S.I., *et al., J. Biol. Chem., 264:* 12053-12062 (1983)). Preparation of the probes was conducted in the following manner. Plasmid-containing E. *coli* was added to an LB medium (Gibco) containing 10 µg/mℓ ampicillin sodium salt (Gibco) and cultured overnight at 37°C, and its plasmid was extracted using a plasmid purification unit available from QIAGEN GmbH (Hilden, Germany). The plasmid was cleaved with HindIII (Takara Shuzo Co., Ltd.) for mdr1a and *BamH*1 (Takara Shuzo Co., Ltd.) for mdr1b and then subjected to 2% agarose gel electrophoresis, and bands of the respective probes were cut out from the gel, and the probes were purified with QIAGEN gel extraction kit. Labeling of both the probes with [α-³²P]dCTP (370 MBq/mℓ, Amersham) was conducted using a rediprime DNA labeling system (Amersham, England). The nylon membrane was pre-hybridized at 65°C for 2 hours in a prehybridization solution (6xSSC, 5xDenhardt's solution, sermon sperm DNA (100 µg/mℓ)) and hybridized at 65°C for 16 hours in a hybridization solution (6xSSC, 5×Denhardt's solution, sermon sperm DNA (100 µg/mℓ), *E. coli* DNA (100 µg/mℓ), polyadenylic acid (50 µg/mℓ)) containing the labeled probes. The membrane was washed 3 times with a washing buffer for 10 minutes at room temperature, then exposed to a BAS-imaging plate for 50 hours, and analyzed by BAS-5000 (Fuji Photo Film Co., Ltd.). When the mdr1a probe was used, a band in the same position (5 kbp) as that of *E. coli*-derived mRNA as the control was confirmed. When the mdr1b probe was used, a band in the same position (5 kbp) as that of *E. coli*-derived mRNA as the control was confirmed. Accordingly, it was confirmed that a mdr1a gene product expressed *in vivo* is expressed in the cell lines obtained this time.

### Example 4

### Establishment of Rat Cultured Astrocyte Cell Line:

Brains were removed from transgenic mice (5 animals) to which a large T antigen gene from temperature-sensitive mutant SV40tsA58 had been introduced [Nakahata, N. et *al., Biochim. Biophys. Acta, 1310:* 60-66 (1996)]. Separation and recovery of brain astrocyte cells were conducted by an enzyme method in the following manner. The brains were placed in a containing a separation buffer [122 mM NaCl, 3 mM KCl, 1.4 mM CaCl₂, 1.2 mM MgSO₄, 0.4 mM K₂HPO₄, 25 mM NaHCO₃, 10 mM glucose, 10 mM HEPES (pH 7.4)] which had been sterilized at 121°C for 15 minutes, transferred to a clean bench and washed with the separation buffer. The brains were cut into square pieces with a side of 2 mm or finely divided with scissors, and 1 mℓ of 2.4 U/mℓ disperse solution was added thereto, and subjected to enzyme treatment at 37°C for 10 minutes. After the undigested fragments were removed, the cells were suspended in a medium [100 U/mℓ penicillin G, 100 mg/mℓ streptomycin sulfate, 10% fetal bovine serum, 9.6 g Dulbecco modified Eagle medium (DMEM), filled up to 1 ℓ by adding distilled water twice].

The cell suspension thus obtained was cultured in a culture dish for 2 days in a 5% CO₂ incubator at 37°C On the 3rd day, the cells were transferred to a 5% CO₂ incubator at 33°C and culture was continued. For subculture, the cells were treated with 0.1% collagenase/disperse solution at 37°C for 5 minutes, allowed to stand at 4°C for 2 hours, and removed from the culture dish.

Cloning of the cells was conducted by a colony formation method in the following manner. The cells after subculture for 1 to 3 generations were recovered, and 10² to 10³ cells were plated onto a 90 mm culture dish to form a colony. The medium was removed under suction, and a cloning cup with grease applied to one side was set up in the culture dish such that the colony was placed at the center. To the cup only, 0.25% trypsin-EDTA solution was added, and the cells were incubated at 33°C for 1 minute. The medium was added to the cup, and the cells were removed by pipetting and cultured in a culture dish. This operation was conducted twice to give cell lines. The cell lines proliferated with a doubling time of about 24 hours and even if subculture progressed till about 30 generations, the cells were stable without any significant alternation in their proliferation ability. They were each designated TR-AST32, TR-AST811, TR-AST912, TR-AST932, and TR-AST943.

TR-AST932 has been deposited as FEPM BP-6283 since March 3, 1998 under the Budapest Treaty with National Institute of Bioscience and Human-Technology, Agency of Industrial Science and Technology (1-3, Higashi 1-chome, Tsukuba-shi, Ibaraki, 305-8566, Japan.

### Example 5

### Expression of GFAP by Established Astrocyte Cell Line and Ability of Transporting L-Glutamic Acid

Expression of glial fibrillary acidic protein (GFAP) as a protein unique to astrocytes by the cells lines obtained in Example 4, that is, TR-AST32, TR-AST811, TR-AST912, TR-AST932 and TR-AST943, was examined by immunostaining. The cells were cultured on a cover glass and used as a sample. The cells were fixed in a PLP solution (sodium perchlorate/L-lysine hydrochloride/paraformaldehyde mixture) under cooling on ice for 15 minutes and blocked with a blocking solution at room temperature for 60 minutes. After 100-fold diluted anti-GFAP antibody was allowed to act on the sample at room temperature for 60 minutes, the sample was washed 3 times with a rinse buffer, and 500-fold diluted peroxidase-labeled anti-rabbit IgG was allowed to act thereon for 60 minutes. After the sample was washed sufficiently with a rinse buffer, coloration reaction was performed. An ice-cold coloration solution was added thereto, and the sample was observed under a microscope, and when the sample was colored, the reaction was terminated by adding cold PBS, and at this stage, the strength of coloration was compared with that of a negative control. As the coloration reagent, 3,3-diaminobenzidine-PBS solution containing 30% hydrogen peroxide was used. As a result, positive staining indicating the expression of GFAP was confirmed in any of TR-AST32, TR-AST811, TR-AST912, TR-AST932 and TR-AST943. Accordingly, it was conformed that TR-AST32, TR-AST811, TR-AST912, TR-AST932 and TR-AST943 are astrocyte cell lines.

The glutamic acid transport ability of the cell lines obtained in Example 4, that is, TR-AST32, TR-AST811, TR-AST912, TR-AST932, TR-AST943 was examined by incorporation of [³H]L-glutamic acid ([³H]L-Glu). Each TR-AST's was plated onto a 24-well cell culture plate at a density of 2×10⁵ cells/well and cultured in a CO₂ incubator at 33°C to make the cells confluent. The incorporation of [³H]L-Glu was measured in the following manner. First, the medium was suctioned, and the cells were washed with an uptake buffer at 37°C, and 0.2 mℓ uptake buffer containing 46.3 kBq/well [³H]L-Glu and 9.25 kBq/mℓ [¹⁴C]inulin at 37°C was added, and the tracer solution was removed after 2, 5, 10 or 30 minutes, and the cells were washed 3 times with 1 mℓ uptake buffer at 4°C. To the cells, 0.75 mℓ of 1% Triton X-100 was added, and the cells were allowed to stand overnight so that they were lyzed and then the lysate was measured for radioactivity in a liquid scintillation counter.

The Km and Vmax for incorporation of 3-OMG were analyzed using a non-linear minimum square program [Yamaoka, K. *et al., J. Pharmacobio-Dyn, 4,* 879-885 (1981)] using a plot formula (V = Vmax × [S]/(Km + [S]); Vmax is a maximum rate constant, Km is a Michaelis constant, and [S] is a substrate concentration) for the rate of incorporation of L-Glu. The results indicated that the incorporation of [³H]L-Glu as the substrate of L-glutamic acid transporter was concentration-dependent, the Michaelis constant (Km) was 96 µM, and the maximum incorporation rate constant (Vmax) was 1.8 nmol/min/mg protein. This incorporation was significantly inhibited by Na⁺-free buffer, and also significantly inhibited by other substrates L-aspartic acid and D-aspartic acid. The results are shown in Table 2.

**Table 2**

| Control | Na⁺ free | 1 mM L-Glu | 1 mM L-Asp | 1 mM D-Asp |
|---|---|---|---|---|
| 100±5 | 68±2 | 8.3±1.5 | 57±2 | 67±6 |
| Expressed in % relative to the control. | | | | |

Accordingly, it was confirmed that the cell lines of the present invention maintained the original function of astrocytes.

As described above, the present invention provides a cell line of brain capillary endothelial cells constituting the blood-brain barrier or a cell line having the properties of astrocyte cells.

While the invention has been described in detail and with reference to specific examples thereof, it will be apparent to one skilled in the art that various changes and modifications can be made therein without departing from the spirit and scope thereof.

## Claims

1. An isolated mammalian brain capillary endothelial cell line having the ability of expressing Na⁺ independent glucose transport carrier (GLUT-1) and/or P-glycoprotein (mdr1a gene product).

2. The cell line according to claim 1, which is derived from a transgenic mouse to which a large T antigen gene from temperature-sensitive mutant SV40tsA58 has been introduced.

3. The cell line according to claim 1 or 2, wherein the cell line is the cell line TM-BBB4 deposited as FERM BP-6282 or the cell lines TM-BBB1, TM-BBB2, TM-BBB3 or TM-BBB5 as described in Example 1.

4. An astrocyte cell line which improves GLUT-1 expression by the brain capillary endothelial cell when co-cultured with a brain capillary endothelial cell according to any one of claims 1 to 3.

5. The astrocyte cell line according to claim 4, which expresses N⁺ dependent L-glutamic acid transporter.

6. The astrocyte cell line according to claim 4 or 5, which is derived from a transgenic rat to which a large T antigen gene of temperature-sensitive mutant SV40tsA58 has been introduced.

7. The astrocyte cell line according to any one of claims 4 to 6, wherein the cell line is the cell line TR-AST932 deposited as FERM BP-6283 or the cell lines TR-AST32, TR-AST811, TR-AST912 or TR-AST943 as described in Example 4.

8. Use of a cell line of any one of claims 1 to 7 for
(a) screening of drugs to identify centrally acting drugs;
(b) screening of drugs to eliminate drugs having the problem of side effects in the nerve center;
(c) screening of drugs targeting various receptors expressed in brain capillary endothelial cells; or
(d) screening of drugs targeting cytopathy.

9. A method of producing a pharmaceutical composition comprising
(a) screening of drugs to identify centrally acting drugs; screening of drugs to eliminate drugs having the problem of side effects in the nerve center; screening of drugs targeting various receptors expressed in brain capillary endothelial cells; or screening of drugs targeting cytopathy; and
(b) formulating said drug identified in screening step (a) into a pharmaceutical composition.
